# EUROPEAN PATENT SPECIFICATION

(11) **EP 2 273 933 B1**
(45) Date of publication and mention of the grant of the patent: **23.08.2017**
(21) Application number: 09725321.5
(22) Date of filing: 27.03.2009
(51) Int. Cl.: A61B 17/34, A61M 16/16, B05B 17/06, A61M 15/00

(54) **HUMIDIFICATION IN BREATHING CIRCUITS**
BEFEUCHTUNG IN ATEMKREISLÄUFEN
HUMIDIFICATION DE CIRCUITS RESPIRATOIRES

(30) Priority: 28.03.2008 IE 20080243; 26.09.2008 IE 20080778
(43) Date of publication of application: 19.01.2011
(60) Divisional application: 17181918.8
(73) Proprietor: STAMFORD DEVICES LIMITED, Dangan Galway (IE)
(72) Inventor: CLANCY, Dermot Joseph, Manorhamilton Count Leitrim (IE); DUFFY, Conor, Roscahill Count Galway (IE); GIBBONS, Keith, Doughiska Galway (IE); POWER, John, County Galway (IE); SMITH, Niall, Alloa FK10 IRR (GB)
(74) Representative: O'Brien, John Augustine
(86) International application number: PCT/IE2009/000011
(87) International publication number: WO 2009/118718

(56) References cited:
- WO-A-2004/074791
- US-A- 5 918 593
- US-A- 5 938 117
- US-A- 6 014 970
- US-A1- 2003 196 660
- US-A1- 2006 151 624

## Description

### Introduction

This invention relates to humidification in breathing circuits for intensive care management of mechanically ventilated patients.

During a normal 24 hour period 250-350 ml of water is lost from the respiratory tract.

During normal breathing the upper respiratory tract humidifies and filters the inspired air. This task occurs primarily in the nasopharynx where air is exposed to a large area of highly vascular, moist mucus membrane. On exhalation some of the moisture taken to humidify the air during inspiration is recovered but the balance, the 250-350ml, is replaced from systemic reserves over time. However, following intubation of patients on mechanical ventilation these normal upper airway moisture exchanging structures are bypassed and the burden of moistening the gases is passed to the lower respiratory tract, which is not suited to this task

A ventilator is used to mechanically move breathable air into and out of patients lungs. For patients who have a long term dependence on a ventilator an endotracheal tube (ET) is passed directly into the patient's trachea in order to ensure that air is able to reach the lungs. The ET is connected by tubing to a Y shaped junction, known as a wye which is connected to the ventilator. One limb of the wye is active during an inspiration phase during which air is delivered into the lungs and the other limb is active during an exhalation phase during which exhaled air is expelled from the lungs.

Heat and moisture exchange (HME) devices are often placed in breathing circuits. They extract heat and moisture from humidified gas exhaled by a patient during the exhalation phase and use the extracted heat and moisture to humidify the dry inspiration gas from the ventilator.

In passive HME devices exhaled heat and humidity is absorbed and transferred to inhaled gas. Such passive heat and moisture exchange systems generally at best only recover 70% of exhaled humidity, creating a humidity deficit, which can create thick obstructive secretions and inflammatory airway reactions in patients with chronic airways disease. Consequently, passive HME is largely confined to use with patients with relatively healthy lungs.

It is also known to augment passive HME devices with the addition of heat and water to boost inhaled absolute humidity. In one known system there is direct application of water to the HME element, and heat is applied to the HME element. In another known system water is heated and vapour passes through a Goretex membrane between the HME device and the patient airway.

In a third known system an active heated humidifier is used in which heat is applied to a body of water, and gas from the ventilator is passed over or through the water as the gas passes along the circuit to the patient, adding water vapour to the gas. Such systems may include heated wires in the inspiratory limb of the ventilator tubing to add heat, or reduce heat loss of gas in transit to the patient.

Some of the problems with known active heated water humidifier systems are that the heated humidifier is bulky, heavy and must be placed close to the ventilator distal to the patient. In addition, there is a likelihood of rain-out down the ventilator circuit tubing which can block the tubing, saturate the exhalation filter or flood the patient. As heated humidified gas passes through the inspiratory limb of the ventilator circuit, it cools and water condenses in the tubing. This condensate can obstruct the airway, interfere with ventilation and/or increase the growth of pathogens. These problems can be mitigated by heating the condensate with heated wires but such heated wire systems add to cost and complexity.

In summary active HME systems add expense with only marginal benefits in humidity. In addition, they add bulk and weight at the airway.

This invention is directed towards providing humidification in breathing circuits which will address at least some of these problems.

US20030196660A describes a ventilator circuit including a nebuliser.

### Statements of Invention

The invention is defined in the claims.

In one embodiment the ventilator circuit comprises an endotracheal tube, an inspiration line extending from a ventilator and an exhalation line extending from the ventilator.

The method may comprise the step of controlling the aerosolisation. Accurate control may be achieved via high speed pulsing of the electrical signal to the aerosol generator. Accurate changes to the pulse frequency will produce accurate and repeatable changes in aerosol output.

Aerosolisation may be controlled responsive to the flow of ventilation gas in the inspiration line. Greater volumes of gas require proportionally greater fluid flow rate of aerosolised humidifying agent to achieve 100% saturation at a given temperature. The gas flow is measured (via flow or differential pressure sensor) in order to determine and deliver the required volume of humidifying agent to achieve 100% RH. Rapid and accurate measurement of gas flow enables required responsiveness.

The method may comprise controlling the fluid flow rate of the aerosolised humidifying agent.

In one embodiment the method comprises the step of determining the flow rate of ventilation gas in the inspiration line.

In one case the method comprises the step of determining if the humidifying agent is in contact with an aerosol generator. This may involve determining at least one electrical characteristic of the aerosol generator. In one case at least electrical characteristics of the aerosol generator over a range of vibration frequencies is determined.

The method may comprise s the step of comparing the at least one electrical characteristic against a pre-defined set of data.

In one case a humidity meter is included close to the patient to measure the level of humidification of the gas entering the body. In this case a feedback loop to the controller is possible to control output from the nebulizer so as to ensure sufficient humidity is present in the ventilator gas.

In another embodiment the ventilator circuit comprises an inspiration line and an exhalation line which are connected at a junction, and a patient line extending from the junction for connection to an endotracheal tube, and the method comprises the step of delivering the aerosolised humidifying agent into the patient line between the junction and the endotracheal tube.

In one case the ventilation circuit comprises a heat and moisture exchange unit in the patient line and the method comprises the step of delivering the aerosolised humidifying agent into the patient line between the heat and moisture exchange unit and the endotracheal tube.

The ventilation circuit can comprise a heat and moisture exchange unit in the patient line and the method comprises delivering the aerosolised humidifying agent into the heat and moisture exchange unit (HME).

In one arrangement a nebuliser is used to provide humidity at patient side of HME in order to replace humidity losses to allow patients use the HME for longer periods of time.

Alternatively a nebuliser may be integrated with HME ('HME booster') providing humidity to a ventilated patient for longer periods of time possible with conventional HME.

In one embodiment the inspiratory gas is heated by providing a heating means such as a heated wire with the nebulizer providing the humidity. In this case a separate HME or hot pot would not be required.

In one embodiment the aerosolised humidifying agent is delivered into the heat and moisture exchange unit on the patient side of the heat and moisture exchange unit.

In one case the aerosol generator is mounted to the heat and moisture exchange unit.

In one embodiment the aerosol generator is integral with the heat and moisture exchange unit.

The invention also provides an aerosol introducer for introducing aerosolised humidifying agent into a ventilation circuit comprising an endotracheal tube, an inspiration line extending from a ventilator, and an exhalation line extending from the ventilator, the introducer comprising an aerosol generator and control means for controlling the operation of the aerosol generator wherein the aerosol generator comprises a vibratable member having a plurality of apertures extending between a first surface and a second surface thereof.

In one case the ventilator circuit comprises an inspiration line and an exhalation line which are connected at a junction, and a patient line extending from the junction comprising e.g. an endotracheal tube, and the method comprises the step of locating the humidifying agent between the junction and the endotracheal tube, or in the inspiratory line prior to the junction.

In one embodiment the controller is configured to control operation of the aerosol generator responsive to the flow of gas in the inspiration line.

In one case the controller is configured to control the flow rate of the humidifying agent to be aerosolised.

In one embodiment the apparatus comprises a device to determine the fluid flow rate of the gas in the inspiration line. The determining device may comprise a flow rate sensor.

In one embodiment the ventilation circuit comprises a junction for connecting the inspiration line and the exhalation line and a patient line for extending between the junction and the endotracheal tube and wherein the aerosol generator is arranged for delivery of aerosolised humidifying agent into the patient line between the junction and the endotracheal tube.

The ventilation circuit may comprise a heat and moisture exchange unit for location in the patient line.

In one arrangement the aerosol generator is arranged for delivery of aerosolised humidifying agent into the patient line between the heat and moisture exchange unit and the endotracheal tube.

The aerosol generator may be mounted on a connector for connection in the patient line.

In one case the aerosol generator is mounted to the heat and moisture exchange unit.

In one embodiment the aerosol generator is integral with the heat and moisture exchange unit.

In one case the first surface is adapted to receive the humidifying agent to be aerosolised.

The aerosol generator may be configured to generate an aerosol at the second surface.

In one case the vibratable member is dome-shaped in geometry. It may also be flat with appropriate stretching.

The vibratable member may comprise a piezoelectric element.

In one case the apertures in the vibratable member are sized to aerosolise the humidifying agent by ejecting droplets of the water such that the majority of the droplets by mass have a size of less than 5 micrometers.

The apertures in the vibratable member can be sized to aerosolise the humidifying agent by ejecting droplets of the water such that the majority of the droplets by mass have a size of less than 3 micrometers.

In one case the controller is configured to control the pulse rate at a set frequency of vibration of the vibratable member.

In one embodiment the controller is impedance matched to the aerosol generator.

In another embodiment the apparatus comprises means to determine whether the humidifying agent is in contact with the aerosol generator.

The determining means may be configured to determine at least one electrical characteristic of the aerosol generator.

The determining means can be configured to determine at least one electrical characteristic of the aerosol generator over a range of vibration frequencies.

In one case the determining means is configured to compare the at least one electrical characteristic against a pre-defined set of data.

### Brief Description of the Drawings

The invention will be more clearly understood from the following description of some embodiments thereof, given by way of example only, with reference to the accompanying drawings, in which: -
Fig. 1A is a diagram of a delivery system according to the invention;
Fig. 1B is a perspective view of an apparatus for humidifying gas in a ventilator circuit according to the invention;
Fig. 2 is a schematic illustration of a part of an apparatus according to the invention;
Fig. 3 is a schematic illustration of a part of the apparatus of Fig. 1;
Fig. 4 is an exploded isometric view of an aerosol generator used in the invention;
Fig. 5 is a cross-sectional view of the assembled aerosol generator of Fig. 4;
Fig. 6 is a perspective view of a controller housing used in the apparatus of the invention;
Figs. 7(a) and 7(b) are graphs of DC voltage versus time and AC voltage versus time respectively to achieve a 100% aerosol output;
Figs. 8(a) and 8(b) are graphs of DC voltage versus time and AC voltage versus time respectively to achieve a 50% aerosol output - Fig 8(a) illustrates the waveform output from a microprocessor to a drive circuit and Fig 8(b) illustrates the waveform output from a drive circuit to a nebuliser;
Figs. 9(a) and 9(b) are graphs of DC voltage versus time and AC voltage versus time respectively to achieve a 25% aerosol output - Fig 9(a) illustrates the waveform output from a microprocessor to a drive circuit and Fig 9(b) illustrates the waveform output from a drive circuit to a nebuliser;
Fig 10 is a graph of AC voltage versus time; and illustrates an output waveform from a drive circuit to a nebuliser;
Fig. 11 is a graph of frequency versus current for another apparatus according to the invention:
Fig. 12 is a perspective view of another apparatus;
Fig. 13 is a cross sectional view of another apparatus;
Fig. 14 is a perspective view of another apparatus of the invention; and
Fig. 15 is a perspective view of another apparatus.

### Detailed Description

The invention provides a method and an apparatus for humidifying gas in a ventilator circuit. In the invention a humidifying agent (sterile water or sterile saline) is aerosolised and then delivered to a ventilator circuit coupled to the respiratory system of a patient.

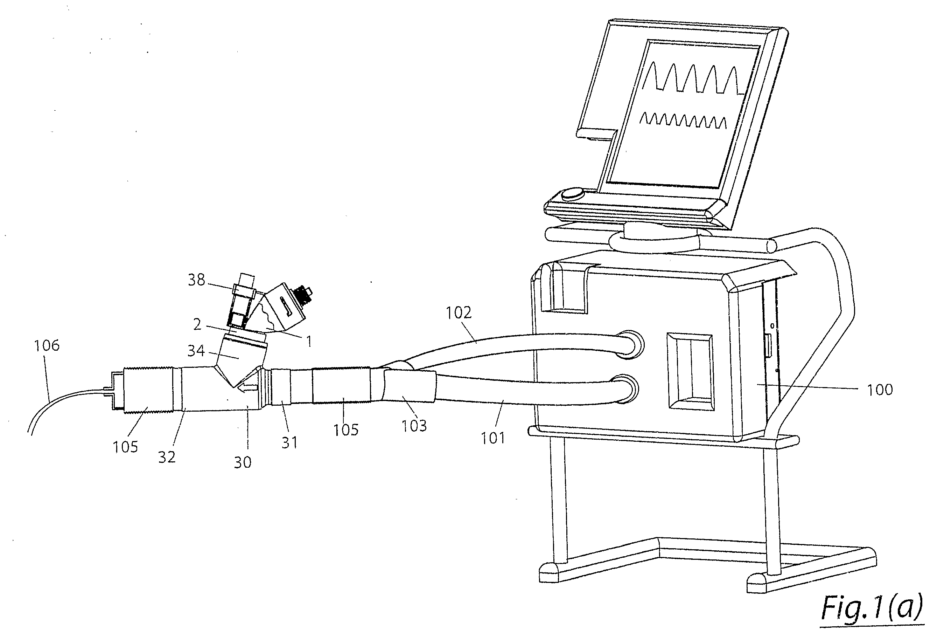

The humidifying system of the invention is particularly useful in delivering the aerosolised humidifying agent to a patient whose breathing is being assisted by a ventilator 100 as illustrated diagrammatically in Fig. 1A. An inhalation or inspiration line 101 extends from the ventilator 100. A return or exhalation line 102 also extends to the ventilator 100. The inspiration and exhalation lines are connected to a junction piece 103, which may be a wye junction. A patient line 105 extends from the wye 103 to an endotracheal tube 106 which extends to the patients lungs. Generally, the various lines 101, 102, 105, 106 are provided by lengths of plastic tubing which are interconnected. The tubing defines lumens for passage of ventilation air, during the inspiration phase, along the inspiration line 101, patient lines 105 and endotracheal tubes 106 into the patients lungs. During the expiration phase exhaled air is delivered along the endotracheal tube 106, patient lines 105 and the expiration line 102. The wye junction 103 provides a common pathway for inspiration and exhalation between the junction 103 and the patients lungs. The ventilator 100 mechanically assists the flow of oxygenated air to the patient during the inspiration phase and in the exhalation phase a patient exhales, either naturally or by the ventilator applying negative pressure.

The apparatus comprises a reservoir 1 for storing sterile water or saline solution, the aerosol generator 2 for aerosolising the water, and a controller 3 for controlling the operation of the aerosol generator 2.

In one aspect of the invention, an aerosol generator 2 is used to deliver an aerosolised humidifying agent into the ventilation air during the inspiration phase.

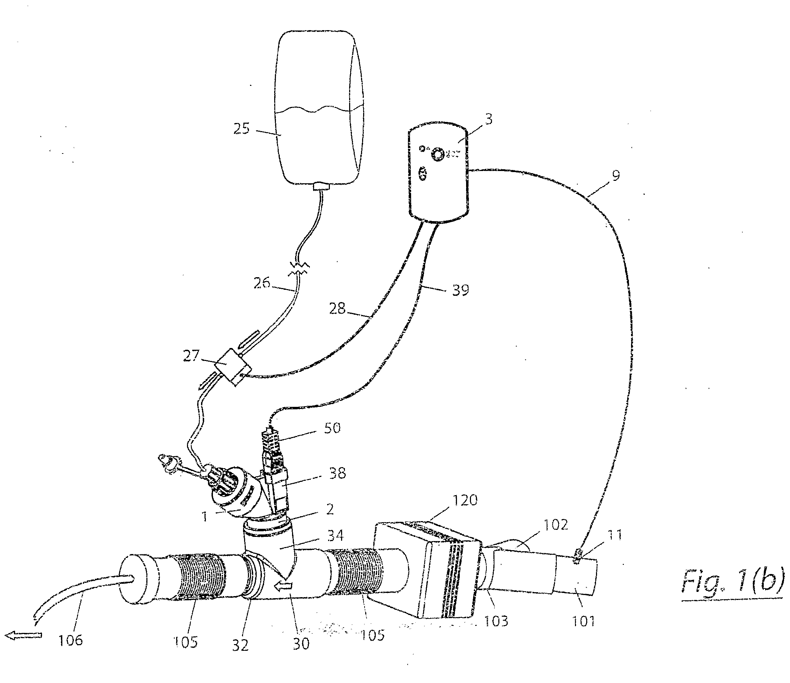

In the arrangement of Fig 1B the apparatus also comprises a sensor 11 for determining flow of air in the inspiration line 10. The sensor 11 is connected by a control wire 9 to the controller 3, and the aerosol generator 2 is also connected to the controller 3.

The humidifying agent may be sterile water or sterile saline with a salt concentration in the range from 1 micromolar to 154 millimolar. Such saline concentrations can be readily nebulised using the aerosolisation technology used in the invention.

In the invention an aerosol is delivered into the breathing circuit. The distinction between aerosol and vapour is in the size of the particles. The majority of aerosol particles that the aerosol generator produces are in the 0.5 to 5.0 micron diameter range. Water vapour on the other hand contains individual water molecules which are approximately 0.00001 microns i.e. 10,000 times smaller than the aerosol particles.

In the invention medical gases for those patients on mechanical ventilation are humidified. The lung is conditioned to receive gas at close to 100% relative humidity (RH) .In the invention when undergoing mechanical ventilation the gas is also at 100% RH when exiting the endotracheal tube.

The amount of water a gas can hold is directly proportional to the temperature of the gas. The table below demonstrates the amount of water that air can hold at various temperatures to give 100% relative humidity

| **Air Temperature °C** | **Amount of H₂O required per L to give 100% RH** |
|---|---|
| 10 | 9.4 mg |
| 20 | 17.4 mg |
| 30 | 30.5 mg |
| 37 | 44.1 mg |

Thus, adding 0.044ml of H₂O to 1L of dry air at 37°Cwill result in the air having a relative humidity of 100%, making it suitable for patients undergoing mechanical ventilation.

This aerosol generator 2 converts the water into an aerosol of a very definable particle size. The volume mean diameter (vmd) would typically be in the range of 2 - 10 microns.

The controller 3 is used to provide electrical power to drive the aerosol generator. This provides the aerosolising action to convey humidification to the breathing circuit.

Referring to Fig. 1A and 1B, in this case an aerosol generator is placed between the Wye 103 and the endotracheal tube (ET) 106 to the patient. The aerosol generator is used to generate an aerosol of sterile water or sterile saline to humidify the gas being delivered to the patient.

In the arrangement of Fig. 1B 100% humidity at the end of the ET tube is achieved by having the nebulizer separate from the HME acting to top-up (boost) the humidity that is lost when using passive humidification via a HME.

In the case of an HME booster the aerosol generator 2 is placed between the ET and a HME 120 as illustrated in Fig.1B.

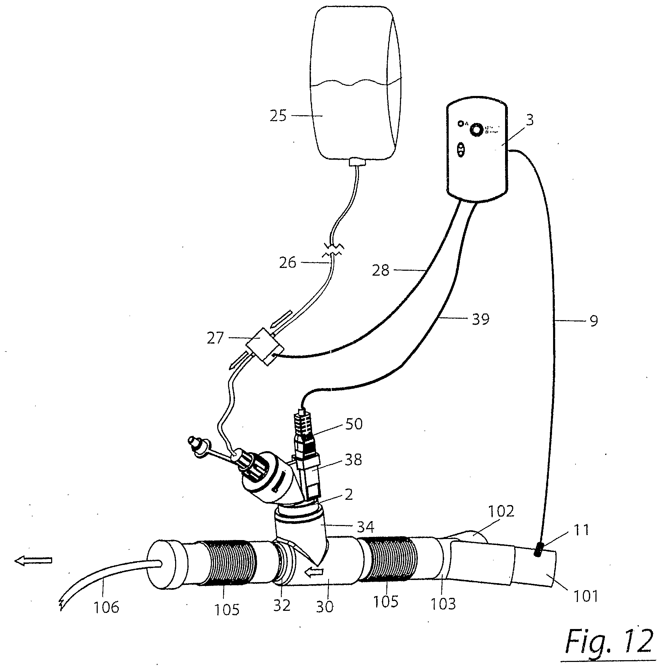

For non boosting applications such as active humidification a HME unit is not required and an aerosol generator 2 is placed between the wye junction 103 and the endotracheal tube 106 as illustrated in Fig. 12. In the arrangement of Fig. 12 100% humidity at the end of the ET tube 106 is achieved because all the humidity for the patient is provided by the nebuliser 2 with no passive humidification.

Aerosol can be delivered continuously, intermittently in short bursts or generated only on inspiration. A flow meter (sensor) 11 may be placed in the inspiratory tubing 101 so that the aerosol output can be adjusted to the inspiratory flow. This provides feedback to the controller 3 to provide aerosol while the sensor 11 detects flow to the patient which occurs in the inhaled breath. Sterile water or sterile normal saline is used as the humidifying agent and the system is sealed from the atmosphere reducing contamination risk.

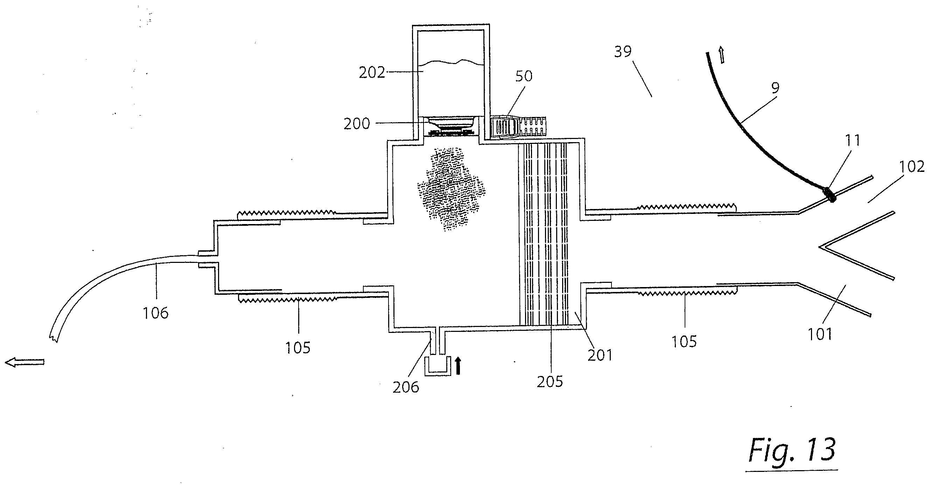

Another variant is illustrated in Fig. 13. This shows a combination of an aerosol generator 200 and a HME (Heat and Moisture Exchange) filter 201 that has an inbuilt liquid reservoir 202. This functions as a booster for passive humidification. In the arrangement of Fig 13 100% humidity at the end of the ET tube is achieved by providing a top-up (boost) in humidity when using passive humidification via the HME, which is provided by an in-built aerosol generator 200 comprising a vibratable aperture plate which is incorporated into the HME. The HME unit 201 has a hydrophobic membrane 205 and is provided with a drain 206. The HME unit also has a drain - off port closable by a lid.

The in built liquid reservoir 202 may be replenished as required, so as to ensure an adequate supply of additional humidity to the circuit.

Aqueous solution may be stored in the reservoir 1 of the nebuliser or the aqueous solution may be delivered to the reservoir 1 of the aerosol generator 2 in this case from a supply reservoir 25 along a delivery line 26. The flow of aqueous solution may be by gravity and/or may be assisted by an in-line flow controlling device 27 such as a pump and/or a valve which may be positioned in the delivery line 26. The operation of the flow controlling device 27 may be controlled by the controller 3 along a control wire 28 to ensure that the aerosol generator 2 has a supply of aqueous solution during operation and yet does not allow fluid build up which may affect the operation of the aersoliser. The device 27 may be of any suitable type.

The vibrating mesh aerosol generator can work with many types of micro pumps. Flow rates of pumps depend on the application and aerosol output requirements however they are typically in the range of 50 nano litres per minute to 5 millilitres per minute. Such micro pumps can have different means of providing the pumping action and can include membrane pumps, electrohydrodynamic (EHD) pumps, electrokinetic, (EK) pumps, rotary pumps, peristaltic pumps, phase change pumps, and several other types of pumps. Diaphragm pumps that are driven by piezo activation are of particular interest as much of the control circuitry utilised is similar to that used to drive vibrating mesh technology and therefore integration of the circuits is simpler and cheaper to undertake.

The invention allows for the nebulization of liquids with surface tensions lower than water. These solutions are nebulizable but due to the surface tension they leak through the aperture plate when left sitting on it. When dispersed onto the aperture plate in a controlled drop by drop fashion the issue of leakage through the aperture plate will not occur.

The device also facilitates nebulisation of solutions that are prone to frothing. The potential to dispense the solution onto the aperture plate in a controlled fashion will prevent the build up of the solution on the aperture plate and the tendency to froth will be eliminated.

The device will reduce unnecessary exposure of solutions that are prone to oxidation or that are light sensitive.

As the pump feed can be located directly behind the aerosol plate it will remove the current restriction of the liquid feed being dependent and will allow the creation of aerosol through 360°C orientation of the device.

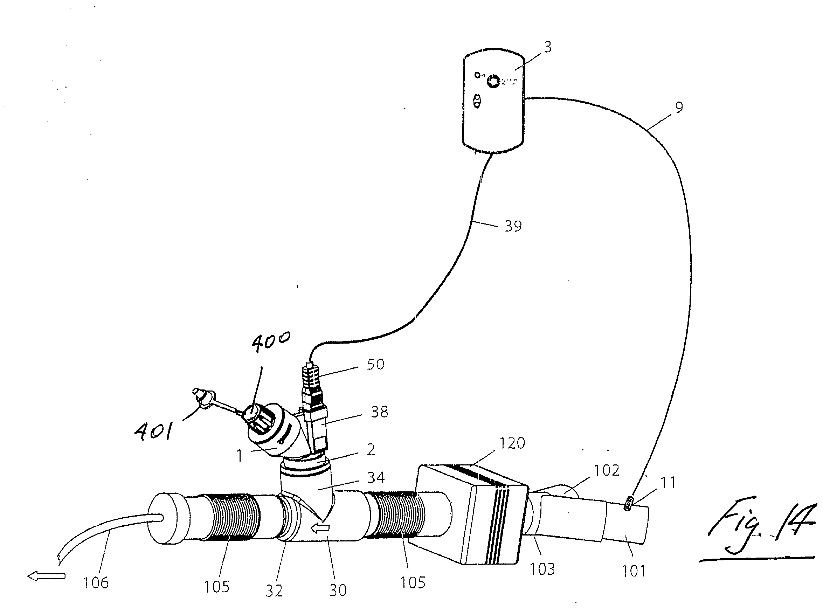

Referring to Fig. 14 there is illustrated another apparatus according to the invention which is similar to that illustrated in Fig. 1B and like parts are assigned the same reference numerals. In this case the nebuliser reservoir 1 has a top opening 400 which is closable by removable plug 401. Liquid, saline or water for humidifying purposes and/or medicament is delivered into the nebuliser reservoir through the opening 400. The opening 400 is appropriately sized to receive standard nebulas containing liquid to be nebulised. The liquid may be applied by syringe or other suitable delivery means.

It is also possible to provide the nebuliser 1 pre-loaded with medicament to avoid the requirement to separately add medicament to the system.

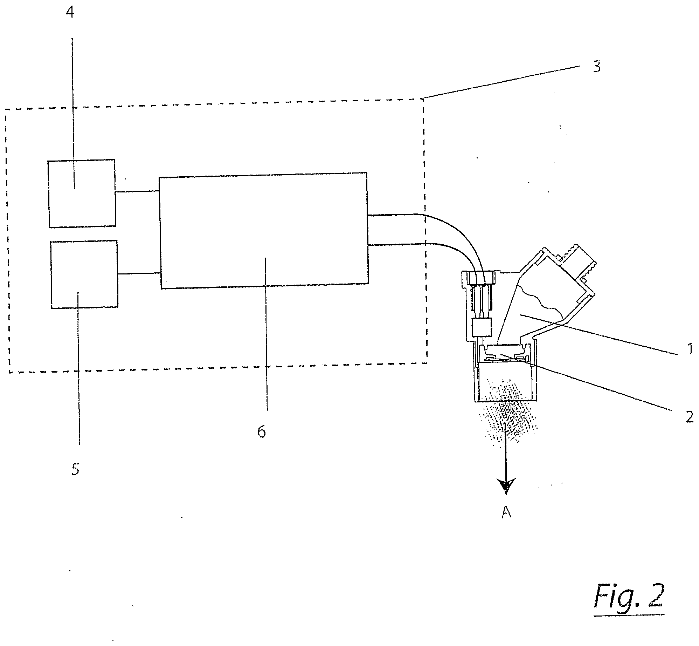

The apparatus of Fig. 14 is operated in a similar way to the modes of operation described with reference to Figs. 2 to 11.

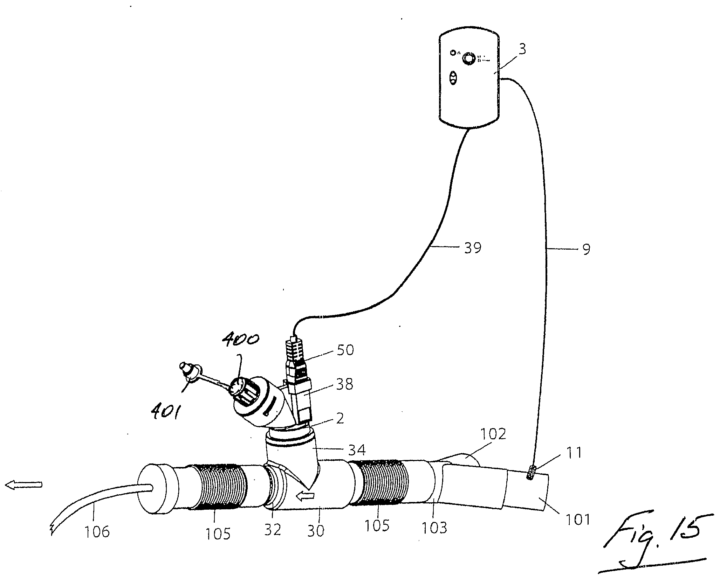

Referring to Fig. 15 there is illustrated another apparatus which is similar to that described above with reference to Fig. 12 and like parts are assigned the same reference numerals. In this case the nebuliser reservoir 1 has a top opening 400 and a removable plug/lid 401 as described with reference to Fig. 14 and the apparatus is operated as described above with the liquid being introduced through the opening 400. Again the nebuliser may be pre-loaded with medicament.

The apparatus comprises a connector 30, in this case a T-piece connector 30 having a ventilation gas conduit inlet 31 and an outlet 32. The connector 30 also comprises an aerosol supply conduit 34 for delivering the aerosol from the aerosol generator 2 into the gas conduit 105 to entrain the aerosol with the ventilation gas, passing through the gas conduit 105. The entrained aerosol/ventilation gas mixture passes out of the connector 30 through the outlet 32 and is delivered to the endotracheal tube 106.

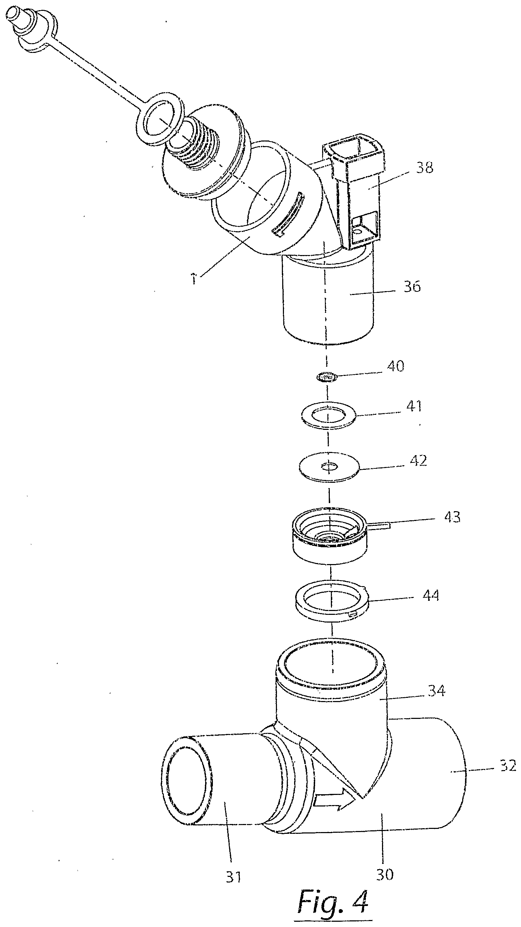

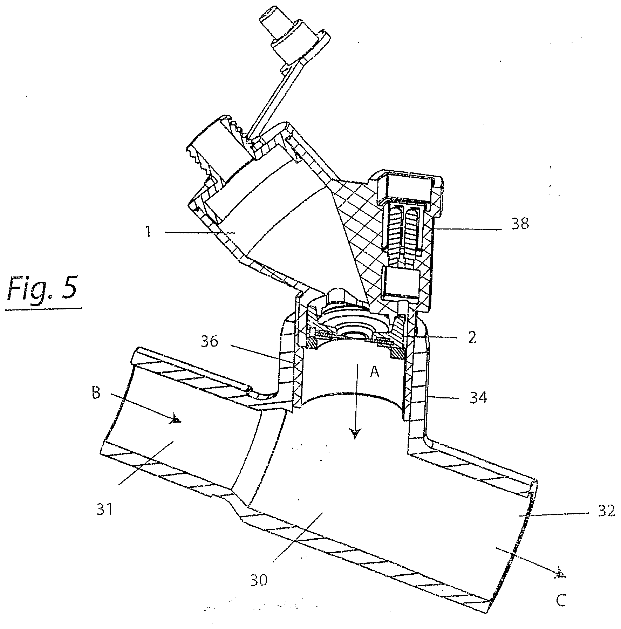

The aerosol supply conduit 34 and the ventilation gas conduit 105 meet at a junction. Referring particularly to Figs. 4 and 5, in the assembled apparatus the aerosol supply conduit of the connector 30 may be releasably mounted to a neck 36 of the aerosol generator housing by means of a push-fit arrangement. This enables the connector 30 to be easily dismounted from the aerosol generator housing 36, for example for cleaning. The neck 36 at least partially lines the interior of the aerosol supply conduit 34.

The nebuliser (or aerosol generator) 2, has a vibratable member which is vibrated at ultrasonic frequencies to produce liquid droplets. Some specific, non-limiting examples of technologies for producing fine liquid droplets is by supplying liquid to an aperture plate having a plurality of tapered apertures extending between a first surface and a second surface thereof and vibrating the aperture plate to eject liquid droplets through the apertures. Such technologies are described generally in U.S. Pat. Nos. 5,164,740; 5,938,117; 5,586,550; 5,758,637; 6,014,970, 6,085,740, and US2005/021 766 A. However, it should be appreciated that the present invention is not limited for use only with such devices.

Various methods of controlling the operation of such nebulisers or aerosol generators are described in US 6,540,154, US 6,845,770, US 5,938,117 and US 6,546,927. In use, the liquid to be aerosolised is received at the first surface, and the aerosol generator 2 generates the aerosolised liquid at the second surface by ejecting droplets of the liquid upon vibration of the vibratable member. The apertures in the vibratable member are sized to aerosolise the liquid by ejecting droplets of the liquid such that the majority of the droplets by mass have a size of less than 5 micrometers.

Referring particularly to Figs 4 and 5, in one case the aerosol generator 2 comprises a vibratable member 40, a piezoelectric element 41 and a washer 42, which are sealed within a silicone overmould 43 and secured in place within the housing 36 using a retaining ring 44. The vibratable member 40 has a plurality of tapered apertures extending between a first surface and a second surface thereof. The first surface of the vibratable member 40, which in use faces upwardly, receives the liquid from the reservoir 1 and the aerosolised liquid, is generated at the second surface of the vibratable member 40 by ejecting droplets of liquid upon vibration of the member 40. In use the second surface faces downwardly. In one case, the apertures in the vibratable member 40 may be sized to produce an aerosol in which the majority of the droplets by weight have a size of less than 5 micrometers.

The vibratable member 40 could be non-planar, and may be dome-shaped in geometry.

The complete nebuliser may be supplied in sterile form, which is a significant advantage for use in breathing circuits.

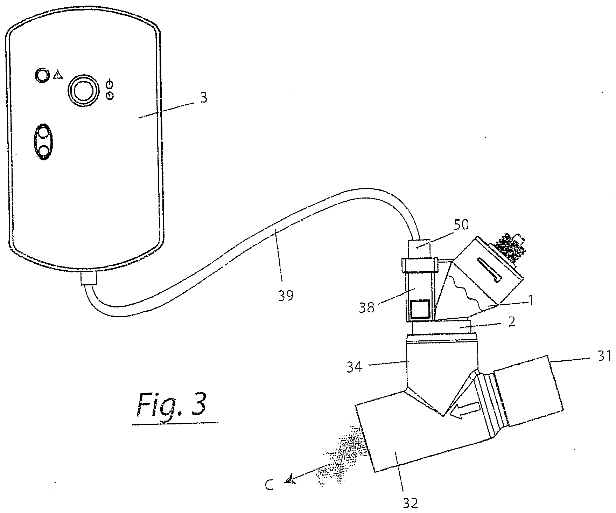

Referring particularly to Fig 3, the controller 3 controls operation of and provides a power supply to the aerosol generator 2. The aerosol generator has a housing which defines the reservoir 1. The housing has a signal interface port 38 fixed to the lower portion of the reservoir 1 to receive a control signal from the controller 3. The controller 3 may be connected to the signal interface port 38 by means of a control lead 39 which has a docking member 50 for mating with the port 38. A control signal and power may be passed from the controller 3 through the lead 39 and the port 38 to the aerosol generator 2 to control the operation of the aerosol generator 2 and to supply power to the aerosol generator 2 respectively.

The power source for the controller 3 may be an on-board power source, such as a rechargeable battery, or a remote power source, such as a mains power source, or an insufflator power source. When the remote power source is an AC mains power source, an AC-DC converter may be connected between the AC power source and the controller 3. A power connection lead may be provided to connect a power socket of the controller 3 with the remote power source.

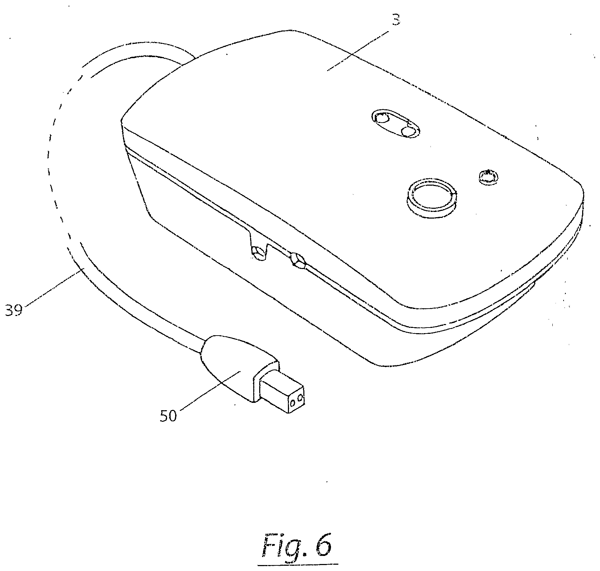

Referring particularly to Fig. 6 the controller 3 has a housing and a user interface to selectively control operation of the aerosol generator 2. Preferably the user interface is provided on the housing which, in use, is located remote from the aerosol generator housing. The user interface may be in the form of, for example, an on-off button. In one embodiment a button can be used to select pre-set values for simplicity of use. In another embodiment a dial mechanism can be used to select from a range of values from 0-100%.

Status indication means are also provided on the housing to indicate the operational state of the aerosol generator 2. For example, the status indication means may be in the form of two visible LED's, with one LED being used to indicate power and the other LED being used to indicate aerosol delivery. Alternatively one LED may be used to indicate an operational state of the aerosol generator 2, and the other LED may be used to indicate a rest state of the aerosol generator. 2.

A fault indicator may also be provided in the form of an LED on the housing. A battery charge indicator in the form of an LED may be provided at the side of the housing.

Referring particularly to Figs 1A and 1B, the liquid in the reservoir 1 flows by gravitational action towards the aerosol generator 2 at the lower medicament outlet. The controller 3 may then be activated to supply power and a control signal to the aerosol generator 2, which causes the piezoelectric element 41 to vibrate the non-planar member 40. This vibration of the non-planar member 40, causes the aqueous solution at the top surface of the member 40 to pass through the apertures to the lower surface where the aqueous solution is aerosolised by the ejection of small droplets of solution.

Referring particularly to Figs 4 and 5, the aerosol passes from the aerosol generator 2 into the neck 36 of the aerosol generator housing, which is mounted within the aerosol supply conduit of the connector 30 and into the gas conduit of the connector 30 (flow A). The aerosol is entrained in the ventilation gas conduit with gas, which passes into the gas conduit through the inlet 31 (flow B). The entrained mixture of the aerosol and the ventilation gas then passes out of the gas conduit through the outlet 32 (flow C) and on to the endotrachael tube 106.

The flow rate sensor/meter 11 determines the flow rate of the ventilation gas. In response to the fluid flow rate of the ventilation gas, the controller 3 commences operation of the aerosol generator 2 to aerosolise the aqueous solution. The aerosolised aqueous solution is entrained with the ventilation gas, and delivered to the patient.

In the event of alteration of the fluid flow rate of the ventilation gas, the flow rate sensor/meter 11 determines the alteration, and the controller 3 alters the pulse rate of the vibratable member of the nebuliser accordingly.

The controller 3 is in communication with the flow rate sensor/meter 11. The controller 3 is configured to control operation of the aerosol generator 2, responsive to the fluid flow rate of the ventilation gas and also independent of the fluid flow rate of the ventilation gas as required.

In one case, the controller 3 is configured to control operation of the aerosol generator 2 by controlling the pulse rate at a set frequency of vibration of the vibratable member, and thus controlling the fluid flow rate of the aqueous solutions.

The controller 3 may comprise a microprocessor 4, a boost circuit 5, and a drive circuit 6. Fig. 2 illustrates the microprocessor 4, the boost circuit 5, the drive circuit 6 comprising impedance matching components (inductor), the nebuliser 2, and the aerosol. The inductor impedance is matched to the impedance of the piezoelectric element of the aerosol generator 2. The microprocessor 4 generates a square waveform of 128KHz which is sent to the drive circuit 6. The boost circuit 5 generates a 12V DC voltage required by the drive circuit 6 from an input of either a 4.5V battery or a 9V AC/DC adapter. The circuit is matched to the impedance of the piezo ceramic element to ensure enhanced energy transfer. A drive frequency of 128 KHz is generated to drive the nebuliser at close to its resonant frequency so that enough amplitude is generated to break off droplets and produce the aerosol. If this frequency is chopped at a lower frequency such that aerosol is generated for a short time and then stopped for a short time this gives good control of the nebuliser's flow rate. This lower frequency is called the pulse rate.

The drive frequency may be started and stopped as required using the microprocessor 4. This allows for control of flow rate by driving the nebuliser 2 for any required pulse rate. The microprocessor 4 may control the on and off times to an accuracy of milliseconds.

The nebuliser 2 may be calibrated at a certain pulse rate by measuring how long it takes to deliver a know quantity of solution. There is a linear relationship between the pulse rate and the nebuliser flow rate. This may allow for accurate control over the delivery rate of the aqueous solution.

The nebuliser drive circuit consists of the electronic components designed to generate output sine waveform of approximately 100V AC which is fed to nebuliser 2 causing aerosol to be generated. The nebuliser drive circuit 6 uses inputs from microprocessor 4 and boost circuit 5 to achieve its output. The circuit is matched to the impedance of the piezo ceramic element to ensure good energy transfer.

The aerosol generator 2 may be configured to operate in a variety of different modes, such as continuous, and/or phasic, and/or optimised..

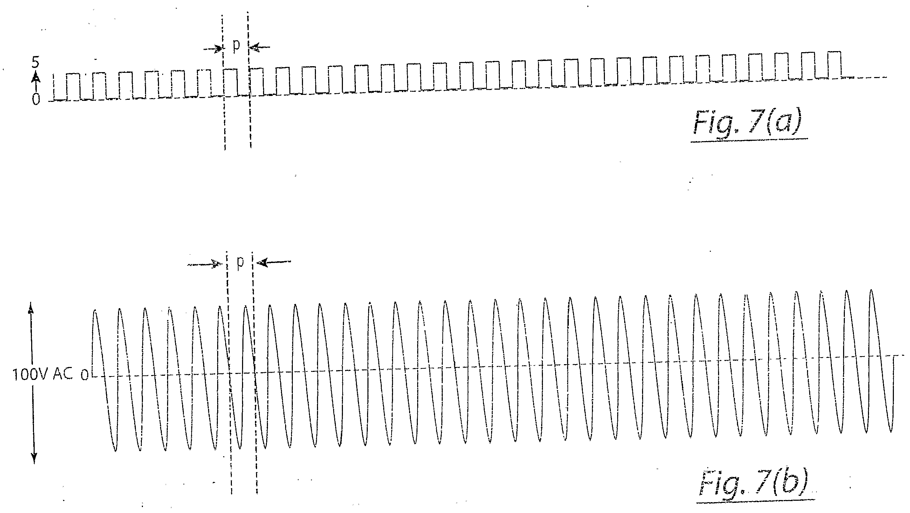

For example, referring to Fig 7(a) illustrates a 5V DC square waveform output from the microprocessor 4 to the drive circuit 6. Fig 7(b) shows a low power, ∼100V AC sine waveform output from drive circuit 6 to nebuliser 2. Both waveforms have a period p of 7.8 µS giving them a frequency of 1/7.8µs which is approximately 128KHz. Both waveforms are continuous without any pulsing. The aerosol generator may be operated in this mode to achieve 100% aerosol output.

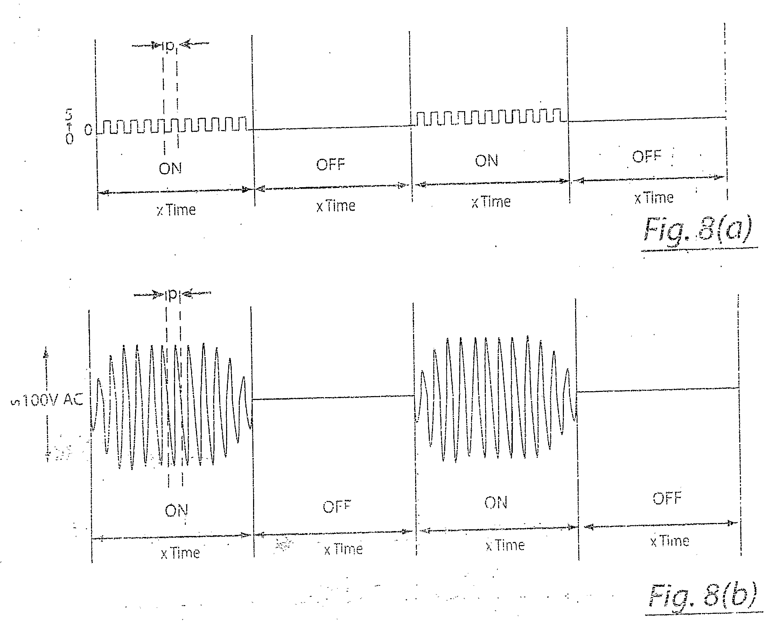

Referring to Figs 8(a) in another example, there is illustrated a 5V DC square waveform output from the microprocessor 4 to the drive circuit 6. Fig 8(b) shows a low power, ∼100V AC sine waveform output from the drive circuit 6 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time x is equal to the on time x. The aerosol generator may be operated in this mode to achieve 50% aerosol output.

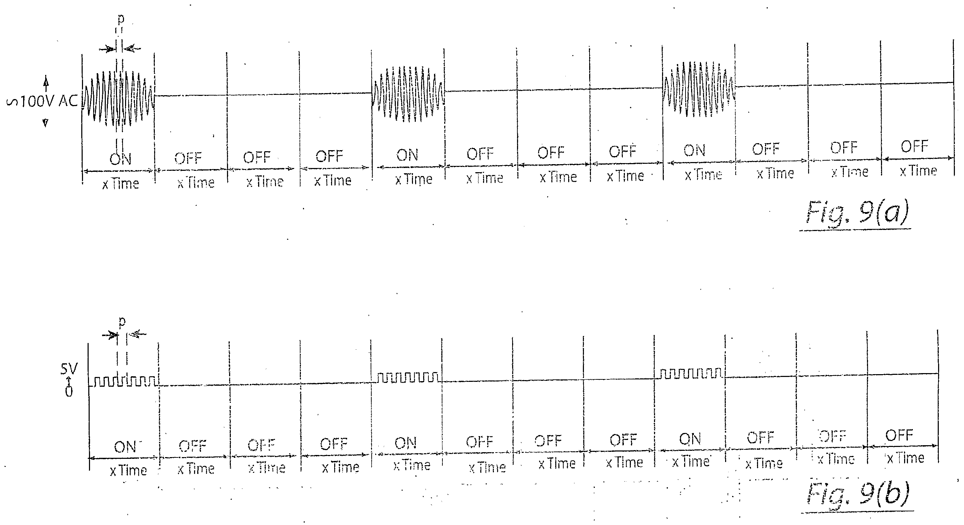

In another case, referring to Figs 9(a) there is illustrated a 5V DC square waveform output from microprocessor 4 to drive circuit 6. Fig 9(b) shows a low power, ∼100V AC sine waveform output from the drive circuit 6 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time is. 3x while the on time is x. The aerosol generator may be operated in this mode to achieve 25% aerosol output.

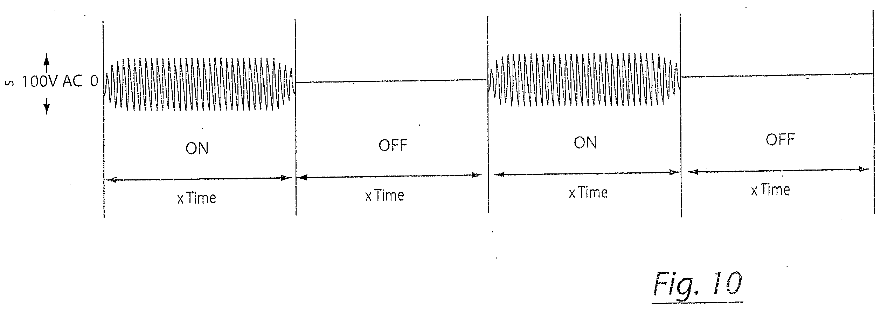

Referring to Fig 10, in one application pulsing is achieved by specifying an on-time and off-time for the vibration of the aperture plate. If the on-time is set to 200 vibrations and off-time is set to 200 vibrations, the pulse rate is 50% (½ on ½ off). This means that the flow rate is half of that of a fully driven aperture plate. Any number of vibrations can be specified but to achieve a linear relationship between flow rate and pulse rate a minimum number of on-time vibrations is specified since it takes a finite amount of time for the aperture plate to reach its maximum amplitude of vibrations.

The drive frequency can be started and stopped as required by the microprocessor; this allows control of flow rate by driving the nebuliser for any required pulse rate. The microprocessor can control the on and off times with an accuracy of microseconds.

A nebuliser can be calibrated at a certain pulse rate by measuring how long it takes to deliver a known quantity of solution. There is a linear relationship between the pulse rate and that nebuliser's flow rate. This allows accurate control of the rate of delivery of the aerosolised aqueous solution.

The pulse rate may be lowered so that the velocity of the emerging aerosol is much reduced so that impaction rain-out is reduced.

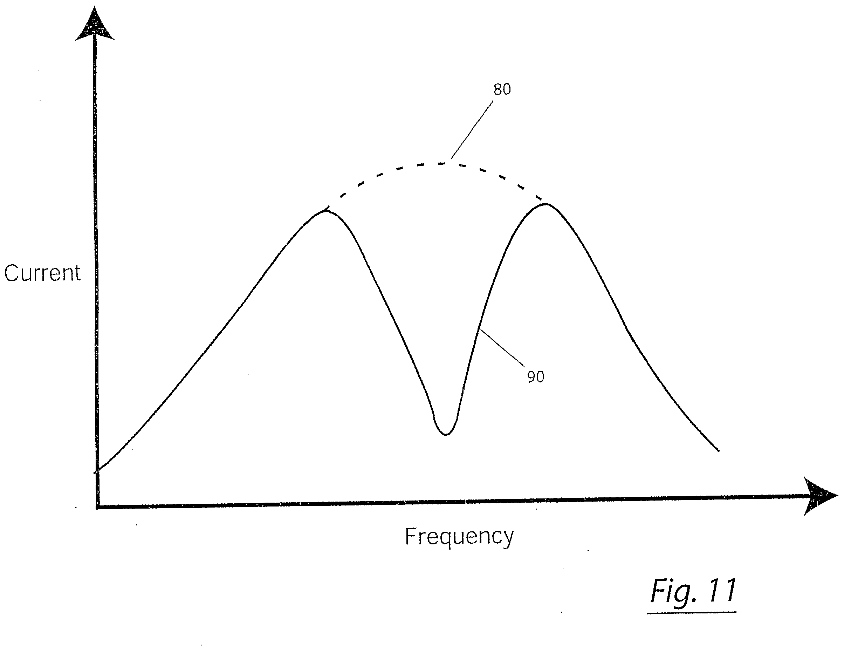

Detection of when the aperture plate is dry can be achieved by using the fact that a dry aperture plate has a well defined resonant frequency. If the drive frequency is swept from 120kHz to 145kHz and the current is measured then if a minimum current is detected less than a set value, the aperture plate must have gone dry. A wet aperture plate has no resonant frequency. The apparatus of the invention may be configured to determine whether there is any of the first fluid in contact with the aerosol generator 2. By determining an electrical characteristic of the aerosol generator 2, for example the current flowing through the aerosol generator 2, over a range of vibration frequencies, and comparing this electrical characteristic against a pre-defined set of data, it is possible to determine whether the aerosol generator 2 has any solution in contact with the aerosol generator 2. Fig. 11 illustrates a curve 80 of frequency versus current when there is some of the solution in contact with the aerosol generator 2, and illustrates a curve 90 of frequency versus current when there is none of the solution in contact with the aerosol generator 2. Fig. 11 illustrates the wet aperture plate curve 80 and the dry aperture plate curve 90.

If an application requires a constant feed from a drip bag then a pump can be added in line to give fine control of the liquid delivery rate which can be nebulised drip by drip. The rate would be set so that liquid would not build up in the nebuliser. This system is particularly suitable for constant low dose delivery.

In the invention the aerosol generator is placed at the patient's endotracheal tube so there is little to no rain - out in the tubing.

The device is very light and unlike the full heated wire system and very silent unlike the jet nebulizer. Non - heated single patient use ventilator tubing can be used. These bring considerable benefits:
▪ reduced cost of maintellance (care giver time)
▪ reduced heat/power cost (in excess of 10 fold)
▪ reduced cost of capital equipment and circuits
▪ reduced background noise

The supply to the aerosol generator is sealed from the atmosphere as it is in a closed circuit and so minimises infection risk even though the aerosol particle size is large enough to carry bacteria.

Intermittent short bursts of aerosol can be programmed to optimize water and heat replenishment of the HME, without requiring more complex aerosol generation patterns.

A drip feed line fed into a small volume reservoir allows the.nebuliser to work in almost any orientation, reducing work and risk for the care giver. This also can provide a very low weight, low profile device.

With the aerosol used to augment the HME, another nebulizer for medication delivery can be placed between the HME and the patient ET, as described for example in US2005/0139211A. The invention can be applied to systems used to ventilate all patients requiring mechanical ventilation or having bypassed upper airways requiring supplemental humidification.

All patients on mechanical ventilation require humidification either with a heated wire humidifier or a heat moisture exchanger. This system can be configured to add supplemental humidity to a HME (heat moisture exchange) only system thereby increasing the capacity of the system to adequately humidify patients for longer periods of time than is possible with current embodiments. The system can also be configured to fully replace the humidification element .of a heated wire humidifier system by adding sufficient amounts of aerosol to the inspired air.

A major problem with the use of nebulizers in the past was contamination of the patient. This has generally been ascribed to the fact that the aerosol particles are of sufficient size to carry bacteria whereas vapour particles are not. The fact that the Aerogen nebulizers can be sterilized and also have the capacity to have a continuous feed of sterile liquid will overcome this reported disadvantage.

The key advantageous features of the invention are:
▪ small/compact
▪ quiet
▪ fed from a sterile sealed system
▪ no large power supply
▪ lower cost
▪ position independent operation

The invention is not limited to the embodiments hereinbefore described, which may be varied in construction and detail.

## Claims

1. A method for humidifying gas in a ventilator circuit comprising:-
aerosolising a humidifying agent using an aerosol generator (2) wherein the aerosol generator (2) comprises a vibratable member (40) having a plurality of apertures extending between a first surface which is adapted to receive the humidifying agent to be aerosolised and a second surface at which an aerosol is generated; and
delivering the aerosolised humidifying agent to a ventilator circuit coupled to the respiratory system of a patient,
wherein the ventilator circuit comprises an inspiration line (101) and an exhalation line (102) which are connected at a junction (103), and a patient line (105) extending from the junction (103) for connection to an endotracheal tube (106), and the method comprises the step of delivering the aerosolised humidifying agent into the patient line between the junction (103) and the endotracheal tube (106), and
**characterised in that** the ventilator circuit comprises a heat and moisture exchange unit (120) in the patient line and the method comprises the step of delivering the aerosolised humidifying agent into the patient line between the heat and moisture exchange unit (120) and the endotracheal tube (106).

2. A method as claimed in claim 1 comprising the step of controlling the aerosolisation.

3. A method as claimed in claim 2 comprising controlling the pulse rate at a set frequency of vibration of the vibratable member (40).

4. A method as claimed in claim 2 or 3 comprising controlling aerosolisation responsive to the flow of ventilation gas in the inspiration line (101).

5. A method as claimed in any of claims 2 to 4 comprising controlling the fluid flow rate of the aerosolised humidifying agent.

6. A method as claimed in claim 4 or 5 wherein the method comprises the step of determining the flow rate of ventilation gas in the inspiration line (101).

7. A method as claimed in claim 6 comprising determining the flow rate of ventilation gas using a flow sensor (11).

8. A method as claimed in claim 6 comprising determining the flow rate of ventilation gas using a differential pressure sensor (11).

9. A method as claimed in any of claims 1 to 8 comprising measuring the level of humidification in the ventilator circuit, downstream of the delivery or aerosolised humidifying agent.

10. A method as claimed in claim 9 comprising controlling the output of the nebuliser (2) responsive to the level of humidification in the ventilator circuit.

11. A method as claimed in any of claims 1 to 10 wherein the method comprises the step of determining if the humidifying agent is in contact with an aerosol generator (2).

12. A method as claimed in claim 11 comprising determining at least one electrical characteristic of the aerosol generator (2).

13. A method as claimed in claim 12 comprising determining at least electrical characteristics of the aerosol generator (2) over a range of vibration frequencies.

14. A method as claimed in claim 11 or 12 wherein the method comprises the step of comparing the at least one electrical characteristic against a pre-defined set of data.

15. A method as claimed in any of claims 1 to 14 comprising heating inspiration gas and delivering the aerosolised humidifying agent into the heated inspiration gas.

16. A ventilator circuit comprising an endotracheal tube (106), an inspiration line (101) extending from a ventilator (100), and an exhalation line (102) extending from the ventilator, and an aerosol introducer apparatus for introducing aerosolised humidifying agent into the ventilator circuit the introducer apparatus comprising an aerosol generator (2) and control means for controlling the operation of the aerosol generator (2) wherein the aerosol generator (2) comprises a vibratable member (40) having a plurality of apertures extending between a first surface which is adapted to receive the humidifying agent to be aerosolised and a second surface at which an aerosol is generated,
wherein the ventilator circuit comprises a junction (103) for connecting the inspiration line (101) and the exhalation line (102) and a patient line (105) for extending between the junction (103) and the endotracheal tube (106),
**characterised in that** the ventilator circuit further comprises a heat and moisture exchange unit (120) for location in the patient line (105), and
**in that** the aerosol generator is arranged for delivery of aerosolised humidifying agent into the patient line (105) between the heat and moisture exchange unit (120) and the endotracheal tube (106).

17. The ventilator circuit as claimed in claim 16 wherein the controller is configured to control operation of the aerosol generator (2) responsive to the flow of gas in the inspiration line (101).

18. The ventilator circuit as claimed in claim 16 or 17 wherein the controller is configured to control the flow rate of the humidifying agent to be aerosolised.

19. The ventilator circuit as claimed in any of claims 16 to 18 wherein the apparatus comprises a device (11) to determine the fluid flow rate of the gas in the inspiration line.

20. The ventilator circuit as claimed in claim 19 wherein the determining device comprises a flow rate sensor (11).

21. The ventilator circuit as claimed in claim 20 wherein the determining device comprises a differential pressure sensor (11).

22. The ventilator circuit as claimed in any of claims 16 to 21 wherein the aerosol generator (2) is mounted on a connector (30) for connection in the patient line.

23. The ventilator circuit as claimed in claim 22 wherein the connector is a T-piece connector (30).

24. The ventilator circuit as claimed in any of claims 16 to 23 comprising heating means (9) for heating inspiration gas.

25. The ventilator circuit as claimed in claim 24 wherein the heating means comprises a heated wire (9) extending along at least a portion of the inspiration line (101).

26. The ventilator circuit as claimed in any of claims 16 to 25 wherein the vibratable member (40) is dome-shaped in geometry.

27. The ventilator circuit as claimed in any of claims 16 to 25 wherein the vibratable member (40) is of stretched flat shape.

28. The ventilator circuit as claimed in any of claims 16 to 27 wherein the vibratable member (40) comprises a piezoelectric element (41).

29. The ventilator circuit as claimed in any of claims 14 to 28 wherein the apertures in the vibratable member (40) are sized to aerosolise the humidifying agent by ejecting droplets of the water such that the majority of the droplets by mass have a size of less than 5 micrometers.

30. The ventilator circuit as claimed in any of claims 16 to 28 wherein the apertures in the vibratable member (40) are sized to aerosolise the humidifying agent by ejecting droplets of the water such that the majority of the droplets by mass have a size of less than 3 micrometers.

31. The ventilator circuit as claimed in any of claims 16 to 28 wherein the apertures in the vibratable member (40) are sized to aerosolise the first fluid by ejecting droplets of the first fluid such that the majority of the droplets by mass have a size range of less than 10 micrometers.

32. The ventilator circuit as claimed in claim 31 wherein a range band is from 1 to 3 micrometers.

33. The ventilator circuit as claimed in claim 31 or 32 wherein a range band is from 7 to 9 micrometers.

34. The ventilator circuit as claimed in any of claims 16 to 33 wherein the controller is configured to control the pulse rate at a set frequency of vibration of the vibratable member (40).

35. The ventilator circuit as claimed in any of claims 16 to 33 wherein the controller is impedance matched to the aerosol generator.

36. The ventilator circuit as claimed in any of claims 16 to 35 wherein the apparatus comprises means to determine whether the humidifying agent is in contact with the aerosol generator (2).

37. The ventilator circuit as claimed in claim 36 wherein the determining means is configured to determine at least one electrical characteristic of the aerosol generator (2).

38. The ventilator circuit as claimed in claim 37 wherein the determining means is configured to determine at least one electrical characteristic of the aerosol generator (2) over a range of vibration frequencies.

39. The ventilator circuit as claimed in claim 37 or 38 wherein the determining means is configured to compare the at least one electrical characteristic against a pre-defined set of data.

40. The ventilator circuit as claimed in any of claims 16 to 39 comprising a flow controlling device (27) for delivery of fluid to be aerosolised to the aerosol generator (2).

41. The ventilator circuit as claimed in claim 40 wherein the flow controlling device comprises a micropump.

42. The ventilator circuit as claimed in claim 41 wherein the micropump comprises a diaphragm pump.

43. The ventilator circuit as claimed in claim 42 wherein the diaphragm pump is driven by piezo activation.

44. The ventilator circuit as claimed in any of claims 40 to 43 wherein the flow controlling device comprises a microvalve.

45. The ventilator circuit as claimed in claim 44 wherein the valve is a solenoid valve.

46. The ventilator circuit as claimed in any of claims 40 to 45 wherein the controller controls the operation of the flow controlling device (27).

47. The ventilator circuit as claimed in any of claims 16 to 46 wherein the aerosol generator comprises a reservoir (1) for liquid to be nebulised and the reservoir has a top opening (400) which is closable by a removable plug (401).

## Patentansprüche

1. Verfahren für die Befeuchtung von Gas in einem Ventilatorkreislauf, das Folgendes umfasst:
Aerosolieren eines Befeuchtungsmittels unter Verwendung eines Aerosolgenerators (2), wobei der Aerosolgenerator (2) ein vibrationsfähiges Element (40) umfasst, das eine Vielzahl von Öffnungen aufweist, die sich zwischen einer ersten Oberfläche, die zur Aufnahme des zu aerosolierenden Befeuchtungsmittels geeignet ist, und einer zweiten Oberfläche, an welcher ein Aerosol erzeugt wird, erstrecken; und
Abgeben des aerosolierten Befeuchtungsmittels zu einem Ventilatorkreislauf, der mit dem Atmungssystem eines Patienten verbunden ist,
wobei der Ventilatorkreislauf Folgendes umfasst: eine Einatmungsleitung (101) und eine Ausatmungsleitung (102), die an einer Anschlussstelle (103) verbunden sind, und eine Patientenleitung (105), die sich von der Anschlussstelle (103) zur Verbindung mit einem Endotrachealtubus (106) erstreckt, und das Verfahren den Schritt der Abgabe des aerosolierten Befeuchtungsmittels in die Patientenleitung zwischen der Anschlussstelle (103) und dem Endotrachealtubus (106) umfasst, und
**dadurch gekennzeichnet, dass** der Ventilatorkreislauf eine Wärme- und Feuchtigkeitsaustauscheinheit (120) in der Patientenleitung umfasst und das Verfahren den Schritt der Abgabe des aerosolierten Befeuchtungsmittels in die Patientenleitung zwischen der Wärme- und Feuchtigkeitsaustauscheinheit (120) und dem Endotrachealtubus (106) umfasst.

2. Verfahren nach Anspruch 1, das den Schritt der Regelung der Aerosolisierung umfasst.

3. Verfahren nach Anspruch 2, das die Regelung der Pulsrate bei einer eingestellten Frequenz der Vibration des vibrationsfähigen Elements (40) umfasst.

4. Verfahren nach Anspruch 2 oder 3, das die Regelung der Aerosolisierung umfasst, die auf den Fluss von Ventilationsgas in die Einatmungsleitung (101) reagiert.

5. Verfahren nach einem der Ansprüche 2 bis 4, das die Regelung der Fluidflussrate des aerosolierten Befeuchtungsmittels umfasst.

6. Verfahren nach Anspruch 4 oder 5, wobei das Verfahren den Schritt der Bestimmung der Flussrate von Ventilationsgas in die Einatmungsleitung (101) umfasst.

7. Verfahren nach Anspruch 6, das die Bestimmung der Flussrate von Ventilationsgas unter Verwendung eines Durchflusssensors (11) umfasst.

8. Verfahren nach Anspruch 6, das die Bestimmung der Flussrate von Ventilationsgas unter Verwendung eines Differenzdrucksensors (11) umfasst.

9. Verfahren nach einem der Ansprüche 1 bis 8, das die Messung der Höhe der Befeuchtung in dem Ventilationskreislauf, der Abgabe des aerosolierten Befeuchtungsmittels nachgeschaltet, umfasst.

10. Verfahren nach Anspruch 9, das die Regelung der Ausgabe des Verneblers (2) umfasst, die auf die Höhe der Befeuchtung in dem Ventilationskreislauf reagiert.

11. Verfahren nach einem der Ansprüche 1 bis 10, wobei das Verfahren den Schritt der Bestimmung umfasst, ob das Befeuchtungsmittel mit einem Aerosolgenerator (2) im Kontakt ist.

12. Verfahren nach Anspruch 11, das die Bestimmung von mindestens einer elektrischen Eigenschaft des Aerosolgenerators (2) umfasst.

13. Verfahren nach Anspruch 12, das die Bestimmung von mindestens elektrischen Eigenschaften des Aerosolgenerators (2) über einen Bereich von Vibrationsfrequenzen umfasst.

14. Verfahren nach Anspruch 11 oder 12, wobei das Verfahren den Schritt des Vergleichs der mindestens einen elektrischen Eigenschaft mit einem vorgegebenen Datensatz umfasst.

15. Verfahren nach einem der Ansprüche 1 bis 14, das die Erwärmung des Einatmungsgases und die Abgabe des aerosolierten Befeuchtungsmittels in das erwärmte Einatmungsgas umfasst.

16. Ventilatorkreislauf, der Folgendes umfasst: einen Endotrachealtubus (106), eine Einatmungsleitung (101), die sich von einem Beatmungsgerät (100) erstreckt, und eine Ausatmungsleitung (102), die sich von dem Beatmungsgerät erstreckt, und eine Aerosoleinführungsvorrichtung für die Einführung von aerosoliertem Befeuchtungsmittel in den Ventilatorkreislauf, wobei die Einführungsvorrichtung einen Aerosolgenerator (2) und Regelungsmittel für die Regelung des Betriebs des Aerosolgenerators (2) umfasst, wobei der Aerosolgenerator (2) ein vibrationsfähiges Element (40) umfasst, das eine Vielzahl von Öffnungen aufweist, die sich zwischen einer ersten Oberfläche, die zur Aufnahme des zu aerosolierenden Befeuchtungsmittels geeignet ist, und einer zweiten Oberfläche, an welcher ein Aerosol erzeugt wird, erstrecken;
wobei der Ventilatorkreislauf Folgendes umfasst: eine Anschlussstelle (103) zur Verbindung der Einatmungsleitung (101) und der Ausatmungsleitung (102) und eine Patientenleitung (105), die sich zwischen der Anschlussstelle (103) und dem Endotrachealtubus (106) erstreckt,
**dadurch gekennzeichnet, dass** der Ventilatorkreislauf weiter eine Wärme- und Feuchtigkeitsaustauscheinheit (120) zur Anordnung in der Patientenleitung (105) umfasst, und
dadurch, dass der Aerosolgenerator für die Abgabe von aerosoliertem Befeuchtungsmittel in die Patientenleitung (105) zwischen der Wärme- und Feuchtigkeitsaustauscheinheit (120) und dem Endotrachealtubus (106) angeordnet ist.

17. Ventilatorkreislauf nach Anspruch 16, wobei der Regler zur Regelung des Betriebs des Aerosolgenerators (2) konfiguriert ist, der auf den Fluss von Gas in der Einatmungsleitung (101) reagiert.

18. Ventilatorkreislauf nach Anspruch 16 oder 17, wobei der Regler zur Regelung der Flussrate des zu aerosolierenden Befeuchtungsmittels konfiguriert ist.

19. Ventilatorkreislauf nach einem der Ansprüche 16 bis 18, wobei die Vorrichtung eine Einrichtung (11) umfasst, um die Fluidflussrate des Gases in der Einatmungsleitung zu bestimmen.

20. Ventilatorkreislauf nach Anspruch 19, wobei die Bestimmungseinrichtung einen Flussratensensor (11) umfasst.

21. Ventilatorkreislauf nach Anspruch 20, wobei die Bestimmungseinrichtung einen Differenzialdrucksensor (11) umfasst.

22. Ventilatorkreislauf nach einem der Ansprüche 16 bis 21, wobei der Aerosolgenerator (2) an ein Verbindungsstück (30) für die Verbindung in der Patientenleitung montiert ist.

23. Ventilatorkreislauf nach Anspruch 22, wobei das Verbindungsstück ein T-Verbindungsstück (30) ist.

24. Ventilatorkreislauf nach einem der Ansprüche 16 bis 23, der Erwärmungsmittel (9) für die Erwärmung von Einatmungsgas umfasst.

25. Ventilatorkreislauf nach Anspruch 24, wobei das Erwärmungsmittel einen Heizdraht (9) umfasst, der sich entlang mindestens eines Teils der Einatmungsleitung (101) erstreckt.

26. Ventilatorkreislauf nach einem der Ansprüche 16 bis 25, wobei das vibrationsfähige Element (40) eine kuppelförmige Geometrie aufweist.

27. Ventilatorkreislauf nach einem der Ansprüche 16 bis 25, wobei das vibrationsfähige Element (40) eine gestreckte flache Form aufweist.

28. Ventilatorkreislauf nach einem der Ansprüche 16 bis 27, wobei das vibrationsfähige Element (40) ein piezoelektrisches Element (41) umfasst.

29. Ventilatorkreislauf nach einem der Ansprüche 14 bis 28, wobei die Öffnungen in dem vibrationsfähigen Element (40) dimensioniert sind, um das Befeuchtungsmittel durch Ausstoß von Tröpfchen des Wassers derart zu aerosolieren, dass die Mehrheit der Tröpfchen massebezogen eine Größe von weniger als 5 Mikrometer aufweist.

30. Ventilatorkreislauf nach einem der Ansprüche 16 bis 28, wobei die Öffnungen in dem vibrationsfähigen Element (40) dimensioniert sind, um das Befeuchtungsmittel durch Ausstoß von Tröpfchen des Wassers derart zu aerosolieren, dass die Mehrheit der Tröpfchen massebezogen eine Größe von weniger als 3 Mikrometer aufweist.

31. Ventilatorkreislauf nach einem der Ansprüche 16 bis 28, wobei die Öffnungen in dem vibrationsfähigen Element (40) dimensioniert sind, um das erste Fluid durch Ausstoß von Tröpfchen des ersten Fluids derart zu aerosolieren, dass die Mehrheit der Tröpfchen massebezogen einen Größenbereich von weniger als 10 Mikrometer aufweist.

32. Ventilatorkreislauf nach Anspruch 31, wobei ein Bereichsband von 1 bis 3 Mikrometer beträgt.

33. Ventilatorkreislauf nach Anspruch 31 oder 32, wobei ein Bereichsband von 7 bis 9 Mikrometer beträgt.

34. Ventilatorkreislauf nach einem der Ansprüche 16 bis 33, wobei der Regler zur Regelung der Pulsrate bei einer eingestellten Frequenz der Vibration des vibrationsfähigen Elements (40) konfiguriert ist.

35. Ventilatorkreislauf nach einem der Ansprüche 16 bis 33, wobei der Regler an den Aerosolgenerator Impedanz-angepasst ist.

36. Ventilatorkreislauf nach einem der Ansprüche 16 bis 35, wobei die Vorrichtung Mittel zur Bestimmung umfasst, ob das Befeuchtungsmittel mit dem Aerosolgenerator (2) im Kontakt ist.

37. Ventilatorkreislauf nach Anspruch 36, wobei das Bestimmungsmittel zur Bestimmung von mindestens einer elektrischen Eigenschaft des Aerosolgenerators (2) konfiguriert ist.

38. Ventilatorkreislauf nach Anspruch 37, wobei das Bestimmungsmittel zur Bestimmung von mindestens einer elektrischen Eigenschaft des Aerosolgenerators (2) über einen Bereich von Vibrationsfrequenzen konfiguriert ist.

39. Ventilatorkreislauf nach Anspruch 37 oder 38, wobei das Bestimmungsmittel zum Vergleich der mindestens einen elektrischen Eigenschaft mit einem vorgegebenen Datensatz konfiguriert ist.

40. Ventilatorkreislauf nach einem der Ansprüche 16 bis 39, der eine Flussregelungseinrichtung (27) für die Abgabe von zu aerosolierendem Fluid an den Aerosolgenerator (2) umfasst.

41. Ventilatorkreislauf nach Anspruch 40, wobei die Flussregelungseinrichtung eine Mikropumpe umfasst.

42. Ventilatorkreislauf nach Anspruch 41, wobei die Mikropumpe eine Membranpumpe umfasst.

43. Ventilatorkreislauf nach Anspruch 42, wobei die Membranpumpe durch Piezo-Aktivierung angetrieben wird.

44. Ventilatorkreislauf nach einem der Ansprüche 40 bis 43, wobei die Flussregelungseinrichtung ein Mikroventil umfasst.

45. Ventilatorkreislauf nach Anspruch 44, wobei das Ventil ein Magnetventil ist.

46. Ventilatorkreislauf nach einem der Ansprüche 40 bis 45, wobei der Regler den Betrieb der Flussregelungseinrichtung (27) regelt.

47. Ventilatorkreislauf nach einem der Ansprüche 16 bis 46, wobei der Aerosolgenerator einen Vorratsbehälter (1) für zu vernebelnde Flüssigkeit umfasst und der Vorratsbehälter eine obere Öffnung (400) aufweist, die durch einen entfernbaren Stopfen (401) verschließbar ist.

## Revendications

1. Procédé d'humidification de gaz dans un circuit de ventilateur comprenant :
la transformation en aérosol d'un agent humectant en utilisant un générateur d'aérosol (2) dans lequel le générateur d'aérosol (2) comprend un élément capable de vibrer (40) ayant une pluralité d'ouvertures s'étendant entre une première surface qui est conçue pour recevoir l'agent humectant devant être transformé en aérosol et une deuxième surface au niveau de laquelle un aérosol est généré ; et
la distribution de l'agent humectant transformé en aérosol vers un circuit de ventilateur couplé au système respiratoire d'un patient,
dans lequel le circuit de ventilateur comprend une ligne d'inspiration (101) et une ligne d'expiration (102) qui sont connectées au niveau d'un branchement (103), et une ligne de patient (105) s'étendant du branchement (103) pour être connectée à un tube endotrachéal (106), et le procédé comprenant l'étape de distribution de l'agent humectant transformé en aérosol jusque dans la ligne de patient entre le branchement (103) et le tube endotrachéal (106), et
**caractérisé en ce que** le circuit de ventilateur comprend une unité échangeuse de chaleur et d'humidité (120) dans la ligne de patient et le procédé comprend l'étape de distribution de l'agent humectant transformé en aérosol jusque dans la ligne de patient entre l'unité échangeuse de chaleur et d'humidité (120) et le tube endotrachéal (106).

2. Procédé selon la revendication 1 comprenant l'étape de régulation de la transformation en aérosol.

3. Procédé selon la revendication 2 comprenant la régulation de la fréquence d'impulsions à une fréquence de vibration réglée de l'élément capable de vibrer (40).

4. Procédé selon la revendication 2 ou 3 comprenant la régulation de la transformation en aérosol en réponse à l'écoulement du gaz de ventilation dans la ligne d'inspiration (101).

5. Procédé selon l'une quelconque des revendications 2 à 4 comprenant la régulation du débit de fluide de l'agent humectant transformé en aérosol.

6. Procédé selon la revendication 4 ou 5 dans lequel le procédé comprend l'étape de détermination du débit du gaz de ventilation dans la ligne d'inspiration (101).

7. Procédé selon la revendication 6 comprenant la détermination du débit du gaz de ventilation en utilisant un capteur d'écoulement (11).

8. Procédé selon la revendication 6 comprenant la détermination du débit du gaz de ventilation en utilisant un capteur de pression différentielle (11).

9. Procédé selon l'une quelconque des revendications 1 à 8 comprenant la mesure du niveau d'humidification dans le circuit de ventilateur, en aval de la distribution de l'agent humectant transformé en aérosol.

10. Procédé selon la revendication 9 comprenant la régulation de la sortie du nébuliseur (2) en réponse au niveau d'humidification dans le circuit de ventilateur.

11. Procédé selon l'une quelconque des revendications 1 à 10 dans lequel le procédé comprend l'étape de détermination si l'agent humectant est en contact avec un générateur d'aérosol (2).

12. Procédé selon la revendication 11 comprenant la détermination d'au moins une caractéristique électrique du générateur d'aérosol (2).

13. Procédé selon la revendication 12 comprenant la détermination au moins de caractéristiques électriques du générateur d'aérosol (2) sur une plage de fréquences de vibration.

14. Procédé selon la revendication 11 ou 12 dans lequel le procédé comprend l'étape de comparaison de cette au moins une caractéristique électrique avec un jeu prédéfini de données.

15. Procédé selon l'une quelconque des revendications 1 à 14 comprenant le chauffage du gaz d'inspiration et la distribution de l'agent humectant transformé en aérosol jusque dans le gaz d'inspiration chauffé.

16. Un circuit de ventilateur comprenant un tube endotrachéal (106), une ligne d'inspiration (101) s'étendant à partir d'un ventilateur (100), et une ligne d'expiration (102) s'étendant à partir du ventilateur, et un appareil introducteur d'aérosol pour introduire l'agent humectant transformé en aérosol jusque dans le circuit de ventilateur, l'appareil introducteur comprenant un générateur d'aérosol (2) et un moyen de régulation pour réguler le fonctionnement du générateur d'aérosol (2) dans lequel le générateur d'aérosol (2) comprend un élément capable de vibrer (40) ayant une pluralité d'ouvertures s'étendant entre une première surface qui est conçue pour recevoir l'agent humectant devant être transformé en aérosol et une deuxième surface au niveau de laquelle un aérosol est généré,
dans lequel le circuit de ventilateur comprend un branchement (103) pour connecter la ligne d'inspiration (101) et la ligne d'expiration (102) et une ligne de patient (105) devant s'étendre entre le branchement (103) et le tube endotrachéal (106),
**caractérisé en ce que** le circuit de ventilateur comprend en outre une unité échangeuse de chaleur et d'humidité (120) devant être située dans la ligne de patient (105), et
**en ce que** le générateur d'aérosol est disposé pour la distribution de l'agent humectant transformé en aérosol jusque dans la ligne de patient (105) entre l'unité échangeuse de chaleur et d'humidité (120) et le tube endotrachéal (106).

17. Circuit de ventilateur selon la revendication 16 dans lequel le régulateur est configuré pour réguler le fonctionnement du générateur d'aérosol (2) en réponse à l'écoulement de gaz dans la ligne d'inspiration (101).

18. Circuit de ventilateur selon la revendication 16 ou 17 dans lequel le régulateur est configuré pour réguler le débit de l'agent humectant devant être transformé en aérosol.

19. Circuit de ventilateur selon l'une quelconque des revendications 16 à 18 dans lequel l'appareil comprend un dispositif (11) pour déterminer le débit de fluide du gaz dans la ligne d'inspiration.

20. Circuit de ventilateur selon la revendication 19 dans lequel le dispositif de détermination comprend un capteur de débit (11).

21. Circuit de ventilateur selon la revendication 20 dans lequel le dispositif de détermination comprend un capteur de pression différentielle (11).

22. Circuit de ventilateur selon l'une quelconque des revendications 16 à 21 dans lequel le générateur d'aérosol (2) est monté sur un connecteur (30) pour une connexion dans la ligne de patient.

23. Circuit de ventilateur selon la revendication 22 dans lequel le connecteur est un connecteur de pièces en T (30).

24. Circuit de ventilateur selon l'une quelconque des revendications 16 à 23 comprenant un moyen de chauffage (9) pour chauffer le gaz d'inspiration.

25. Circuit de ventilateur selon la revendication 24 dans lequel le moyen de chauffage comprend un fil chauffant (9) s'étendant le long d'au moins une partie de la ligne d'inspiration (101).

26. Circuit de ventilateur selon l'une quelconque des revendications 16 à 25 dans lequel l'élément capable de vibrer (40) a la forme géométrique d'un dôme.

27. Circuit de ventilateur selon l'une quelconque des revendications 16 à 25 dans lequel l'élément capable de vibrer (40) est de forme plate étirée.

28. Circuit de ventilateur selon l'une quelconque des revendications 16 à 27 dans lequel l'élément capable de vibrer (40) comprend un élément piézoélectrique (41).

29. Circuit de ventilateur selon l'une quelconque des revendications 14 à 28 dans lequel les ouvertures dans l'élément capable de vibrer (40) sont dimensionnées pour transformer en aérosol l'agent humectant en éjectant des gouttelettes de l'eau de sorte que la majorité des gouttelettes en masse ont une taille de moins de 5 micromètres.

30. Circuit de ventilateur selon l'une quelconque des revendications 16 à 28 dans lequel les ouvertures dans l'élément capable de vibrer (40) sont dimensionnées pour transformer en aérosol l'agent humectant en éjectant des gouttelettes de l'eau de sorte que la majorité des gouttelettes en masse ont une taille de moins de 3 micromètres.

31. Circuit de ventilateur selon l'une quelconque des revendications 16 à 28 dans lequel les ouvertures dans l'élément capable de vibrer (40) sont dimensionnées pour transformer en aérosol le premier fluide en éjectant des gouttelettes du premier fluide de sorte que la majorité des gouttelettes en masse ont une plage de tailles de moins de 10 micromètres.

32. Circuit de ventilateur selon la revendication 31 dans lequel une bande de plage est de 1 à 3 micromètres.

33. Circuit de ventilateur selon la revendication 31 ou 32 dans lequel une bande de plage est de 7 à 9 micromètres.

34. Circuit de ventilateur selon l'une quelconque des revendications 16 à 33 dans lequel le régulateur est configuré pour réguler la fréquence d'impulsions à une fréquence de vibration réglée de l'élément capable de vibrer (40).

35. Circuit de ventilateur selon l'une quelconque des revendications 16 à 33 dans lequel l'impédance du régulateur est adaptée au générateur d'aérosol.

36. Circuit de ventilateur selon l'une quelconque des revendications 16 à 35 dans lequel l'appareil comprend un moyen pour déterminer si l'agent humectant est en contact avec le générateur d'aérosol (2).

37. Circuit de ventilateur selon la revendication 36 dans lequel le moyen de détermination est configuré pour déterminer au moins une caractéristique électrique du générateur d'aérosol (2).

38. Circuit de ventilateur selon la revendication 37 dans lequel le moyen de détermination est configuré pour déterminer au moins une caractéristique électrique du générateur d'aérosol (2) sur une plage de fréquences de vibration.

39. Circuit de ventilateur selon la revendication 37 ou 38 dans lequel le moyen de détermination est configuré pour comparer cette au moins une caractéristique électrique avec un jeu prédéfini de données.

40. Circuit de ventilateur selon l'une quelconque des revendications 16 à 39 comprenant un dispositif de régulation du débit (27) pour distribuer le fluide devant être transformé en aérosol avec le générateur d'aérosol (2).

41. Circuit de ventilateur selon la revendication 40 dans lequel le dispositif de régulation du débit comprend une micropompe.

42. Circuit de ventilateur selon la revendication 41 dans lequel la micropompe comprend une pompe à diaphragme.

43. Circuit de ventilateur selon la revendication 42 dans lequel la pompe à diaphragme est actionnée par activation piézoélectrique.

44. Circuit de ventilateur selon l'une quelconque des revendications 40 à 43 dans lequel le dispositif de régulation du débit comprend une microsoupape.

45. Circuit de ventilateur selon la revendication 44 dans lequel la soupape est une soupape à solénoïde.

46. Circuit de ventilateur selon l'une quelconque des revendications 40 à 45 dans lequel le régulateur régule le fonctionnement du dispositif de régulation du débit (27).

47. Circuit de ventilateur selon l'une quelconque des revendications 16 à 46 dans lequel le générateur d'aérosol comprend un réservoir (1) pour le liquide devant être nébulisé et le réservoir a une ouverture supérieure (400) qui peut être refermée par un bouchon amovible (401).
